# EUROPEAN PATENT APPLICATION

(11) **EP 4 344 625 A1**
(43) Date of publication of application: **03.04.2024**
(21) Application number: 21948568.7
(22) Date of filing: 10.11.2021
(51) Int. Cl.: A61B 5/145, A61B 5/155, A61B 5/15, A61M 5/172, A61M 5/142

(54) **APPLICATOR FOR TRANSCUTANEOUS SENSOR, AND APPLICATOR ASSEMBLY**

(30) Priority: 29.06.2021 KR 20210084623
(71) Applicant: i-Sens, Inc., Seoul 06646 (KR)
(72) Inventor: CHOI, Hyun Ho, Seoul 06646 (KR); RYU, Goang Yel, Seoul 06646 (KR); WANG, Ji Hoon, Seoul 06646 (KR)
(74) Representative: BCKIP Part mbB
(86) International application number: PCT/KR2021/016304
(87) International publication number: WO 2023/277268

(57) **Abstract**

An applicator, according to the present invention, for inserting, into the skin of a user, a sensor for measuring biological information comprises: an applicator body having, detachably coupled thereto, a base unit including a base unit housing and an adhesive part, which is provided on the base unit housing so as to be attachable to the skin of the user; a locking hook provided at the applicator body so as to engage with the base unit in order to maintain the base unit in a state of being coupled to the applicator body; an insertion unit provided at the applicator body and moving, from a first position at which a sensor unit is spaced from the base unit to a second position at which the sensor unit is coupled to the base unit, the sensor unit including the sensor and a sensor unit housing in which the sensor is mounted, in order to insert the sensor into the skin of the user; and a moving member which is provided at the applicator body so as to be movable from a third position to a fourth position, and which skews the locking hook at the fourth position so that same disengages from the base unit.

## Description

### TECHNICAL FIELD

The present disclosure relates to an applicator for a transcutaneous sensor, and more specifically, related to an applicator and an applicator assembly for inserting a transcutaneous sensor, which is inserted into the skin of a user to measure biometric information, into the skin of a user.

### BACKGROUND

With the recent advancement of medical technology, various medical devices that are attached to the body of a user have been developed and sold. Medical devices that are attached to the skin can be useful for monitoring biometric information or providing treatment by attaching them to the body of a patient with a chronic disease.

For example, chronic diseases such as diabetes require continuous management, and a body attachable unit for measuring biometric information can be used to manage blood glucose levels in diabetic patients. Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown. Further progression of diabetes leads to complications such as visual disturbances, hypertension, kidney disease, paralysis, periodontal disease, muscle spasms and neuralgia, as well as gangrene. In order to diagnose diabetes beforehand and manage to prevent the progression of diabetes into complications associated therewith, systematic blood glucose measurement and treatment should be performed.

For diabetes patients as well as people having higher than normal blood glucose, even though diabetes has not yet developed, medical device manufacturers offer a variety of blood glucose meters to measure blood glucose levels.

Glucose measuring devices may be categorized into a single time measurement type measuring a blood glucose level and collecting blood from a fingertip by a user every single time and a continuous measurement type attaching a glucose monitoring system to the belly or an arm of the user and continuously measuring blood glucose levels.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

Recently, to overcome such a drawback, continuous glucose monitoring systems (CGMSs) inserted into the human body to measure a blood glucose level every few minutes have been developed, and therefore easily perform the management of diabetics and responses to an emergency situation.

A continuous blood glucose measurement system includes a body attachable unit that includes a transcutaneous sensor that is inserted into the skin of a user and measures blood glucose levels in the body, and a terminal that receives biometric information transmitted from the body attachable unit and outputs.

Systems for medical purposes using transcutaneous sensors are produced in many different forms by each manufacturer, and the methods of use also vary. Most systems currently manufactured and distributed are a method of attaching a disposable sensor unit to a body by an applicator. A user needs to perform several steps to attach the sensor unit to a skin using an applicator, and after attaching the sensor unit to a body, various follow-up procedures such as having to directly pull out the needle inserted into the skin along with the transcutaneous sensor are required to be performed.

For example, a user must peel off the packaging of the disposable sensor unit and accurately attach it to the applicator, and then insert the sensor unit into the applicator and operate the applicator to attach the sensor unit to a skin. Additionally, after attaching the sensor unit, there is the inconvenience of having to perform tasks such as pulling out the needle inserted into the skin and coupling the transmitter to the sensor unit.

### SUMMARY

### Technical Problem

The present disclosure is developed in consideration of the above-mentioned points, and the purpose of the present disclosure is to minimize the additional work to insert the transcutaneous sensor into a skin by enabling the sensor unit including the transcutaneous sensor to be manufactured in an assembled state, and to provide an applicator and applicator assembly for a transcutaneous sensor that can be conveniently used by a user to insert a transcutaneous sensor into the skin.

Additionally, another object of the present disclosure is to provide an applicator and an applicator assembly with an improved coupling structure between an applicator and a base unit which is detachably coupled to the applicator in a separate state from the sensor unit.

### Solution to Problem

To accomplish the above-described purposes, an applicator for inserting a sensor for measuring biometric information into skin of a user according to an embodiment of the present disclosure may comprise: an applicator body to which a base unit comprising a base unit housing and an adhesive portion provided on the base unit housing to be attached to the skin of the user is separatably coupled; a locking hook installed to the applicator body to be engaged with the base unit to maintain a state in which the base unit is coupled to the applicator body; an insertion unit installed to the applicator body to insert the sensor into the skin of the user by moving the sensor unit, comprising the sensor and a sensor unit housing to which the sensor is mounted, from a first position spaced apart from the base unit to a second position coupled with the base unit; and a moving member installed to the applicator body to be movable from a third position to a fourth position, and configured to bias the locking hook to be disengaged from the base unit at the fourth position.

The moving member may be installed to move in a direction of intersecting a direction in which the sensor unit moves from the first position to the second position.

The insertion unit may comprise a plunger installed to be movable from the first position to the second position together with the sensor unit, a needle separatably coupled with the sensor unit to be inserted into the skin of the user with the sensor, a carrier installed to the plunger and coupled with the needle, and a plunger driver configured to provide a moving force to the plunger in a direction of moving from the first position to the second position, and the plunger may be configured to be restricted from moving at the first position by contacting the moving member located at the third position, and, when the moving member moves to the fourth position, move away from the moving member and move to the second position.

The applicator body may comprise a base frame comprising a base portion configured to be contactable with the skin of the user, and a column portion protruding from the base portion and accommodating the plunger, and a middle frame having a stage disposed at an upper side of the base portion to support the moving member, and a middle frame opening formed in a middle of the stage such that the column portion may be inserted in the middle frame opening.

The applicator according to an embodiment of the present disclosure may further comprise: an elastic member configured to apply an elastic force to the moving member such that the moving member is movable from the third position to the fourth position; a stopper configured to restrain movement of the moving member in a state in which the elastic member is elastically deformed; and an operating member configured to release the stopper such that the moving member is movable by the elastic member according to manipulation of the user.

The locking hook may be pivotally coupled to the applicator body, and the moving member may move from the third position to the fourth position to rotate the locking hook in a direction of disengaging the locking hook from the base unit.

The locking hook may comprises a pivot portion coupled to the applicator body, a hook portion protruding from the pivot portion to be engaged with the base unit, and a rod protruding from the pivot portion to be engaged with the moving member, wherein the moving member is installed to move in a direction of intersecting a direction in which the sensor unit moves from the first position to the second position, and wherein the moving member is provided with a guide portion engaged with the rod in an inclined form with respect to a moving direction of the moving member, and the guide portion moves in a state of being engaged with the rod to rotate the locking hook.

The applicator according to an embodiment of the present disclosure may further comprise a moving member driver configured to move the moving member from the third position to the fourth position.

The moving member driver may include an elastic member configured to apply an elastic force to the moving member.

The applicator according to an embodiment of the present disclosure may further comprise: a stopper configured to restrain movement of the moving member in a state in which the elastic member is elastically deformed; and an operating member configured to release the stopper such that the moving member is movable by the elastic member according to manipulation of the user.

Additionally, to accomplish the above-described purposes, an applicator for inserting a sensor for measuring biometric information into skin of a user according to another embodiment of the present disclosure may comprise: an applicator body to which a body attachable unit base unit comprising the sensor, a base unit housing, and an adhesive portion provided on the base unit housing to be attached to the skin of the user is separatably coupled; a locking hook installed to the applicator body to be engaged with the base unit housing to maintain a state in which the base unit housing is coupled to the applicator body; a needle installed to the applicator body such that the needle is movable from the first position to the second position to be inserted into the skin of the user together with the sensor; and a moving member installed to the applicator body to be movable from a third position to a fourth position, and configured to bias the locking hook to be disengaged from the base unit housing at the fourth position.

In addition, to accomplish the above-described purposes, an applicator assembly according to an embodiment of the present disclosure may comprise: an applicator body configured to be contactable with skin of a user; a sensor unit comprising a sensor and a sensor unit housing to which the sensor is mounted; a base unit including a base unit housing and an adhesive portion provided on the base unit housing to be attached to the skin of the user, disposed to be spaced apart from the sensor unit, and separatably coupled to the applicator body; an insertion unit installed to the applicator body to move the sensor unit from a first position away from the base unit to a second position where the sensor is inserted into the skin of the user to couple the sensor unit with the base unit; a locking hook installed to the applicator body to be engaged with the base unit to maintain a state in which the base unit is coupled to the applicator body; and a moving member installed to the applicator body to be movable from a third position to a fourth position, and configured to bias the locking hook to be disengaged from the base unit at the fourth position.

### Advantageous Effects of Invention

According to the present disclosure, by manufacturing the sensor unit in an assembled state within the applicator, the additional work for a user to attach the sensor unit to the skin of a user is minimized, and the sensor unit can be attached to the skin of a user simply by operating the applicator.

Additionally, according to the present disclosure, before the sensor unit is coupled to the base unit, the base unit is stably fixed to the applicator body so that it is not easily separated from the applicator body, and after the sensor unit is coupled to the base unit, the base unit can be smoothly separated from the applicator body.

### BRIEF DESCRIPTION OF THE DRAWINGS

FIG. 1 is a perspective view showing an applicator assembly according to an embodiment of the present disclosure.
FIG. 2 shows a body attachable unit attached to the skin of a user.
FIG. 3 is a perspective view showing a body attachable unit.
FIG. 4 shows a protective sheet attached to an adhesive layer of a sensor unit.
FIG. 5 shows an appearance after a protective sheet is separated from an adhesive layer of a sensor unit.
FIG. 6 shows a sensor unit and a base unit before they are coupled.
FIG. 7 is a cross-sectional view taken along line I-I in FIG. 1.
FIG. 8 is a cross-sectional view taken along line II-II in FIG. 1.
FIG. 9 is an exploded perspective view of an applicator according to an embodiment of the present disclosure.
FIG. 10 is a perspective view showing a needle assembly of an applicator according to an embodiment of the present disclosure.
FIG. 11 is a perspective view showing a partial configuration of an applicator according to an embodiment of the present disclosure.
FIG. 12 shows a view in which an operating member of an applicator according to one embodiment of the present disclosure biases the stopper to be disengaged from a moving member.
FIG. 13 is a side view showing a moving member of an applicator according to an embodiment of the present disclosure fixed in the third position by a stopper.
FIG. 14 is a plan view showing a moving member of an applicator according to an embodiment of the present disclosure fixed in the third position by a stopper.
FIG. 15 is a side view showing a protective sheet of a sensor unit being separated by moving a moving member of an applicator according to an embodiment of the present disclosure to the fourth position.
FIG. 16 is a plan view showing a moving member of an applicator according to an embodiment of the present disclosure moved to the fourth position.
FIG. 17 shows a locking hook of an applicator according to an embodiment of the present disclosure engaged with a base unit.
FIGS. 18 and 19 show a locking hook of the applicator according to an embodiment of the present disclosure connected to a guide portion of a moving member.
FIGS. 20 to 25 show a process in which a sensor of a sensor unit is inserted into the skin of a user by an applicator according to an embodiment of the present disclosure.

### DETAILED DESCRIPTION OF EMBODIMENTS

Hereinafter, the applicator and applicator assembly for a transcutaneous sensor according to the present disclosure will be described in detail with reference to the drawings.

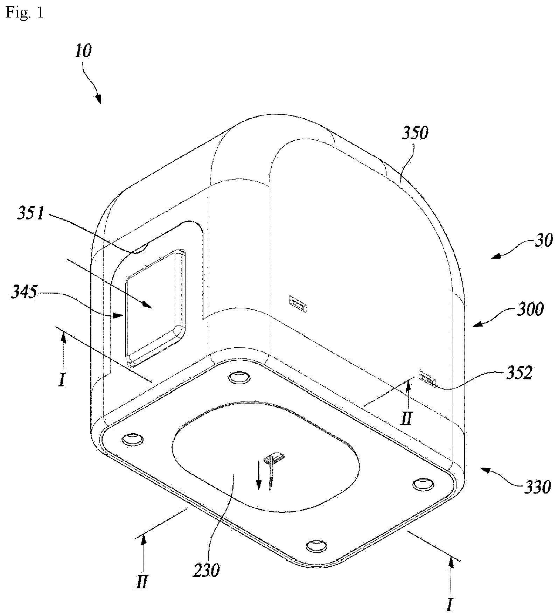

FIG. 1 is a perspective view showing an applicator assembly according to an embodiment of the present disclosure.

An applicator assembly (10) according to an embodiment of the present disclosure includes a sensor unit (100) including a sensor (110) that is inserted into the skin of a user and measures biometric information, and a base unit (200) that is coupled with the sensor unit (100) to form a body attachable unit (20), and an applicator (30) for attaching the body attachable unit (20) to the skin of a user. The applicator assembly (10) may be provided to a user with the sensor unit (100) and the base unit (200) mounted separately on the applicator (30). The sensor unit (100) and the base unit (200) can be automatically assembled and attached to the skin of a user by the operation of the applicator (30).

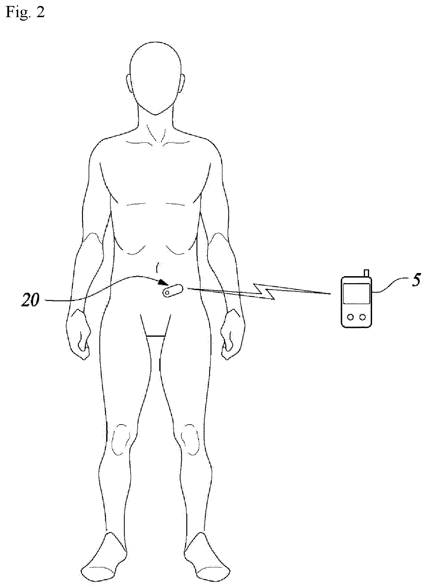

As shown in FIG. 2, the body attachable unit (20) can be attached to the skin of a user by the applicator (30), measure biometric information, and wirelessly transmit the measured data to an external terminal (5). The external terminal (5) may be a variety of devices capable of receiving measurement data from the body attachable unit (20), such as a portable terminal, a medical device, a PC, or a server. Biometric information that the body attachable unit (20) can measure is not limited to specific information. As an exemplary embodiment, the body attachable unit (20) may periodically measure blood glucose level for a user and transmit the blood glucose measurement information to the external terminal (5).

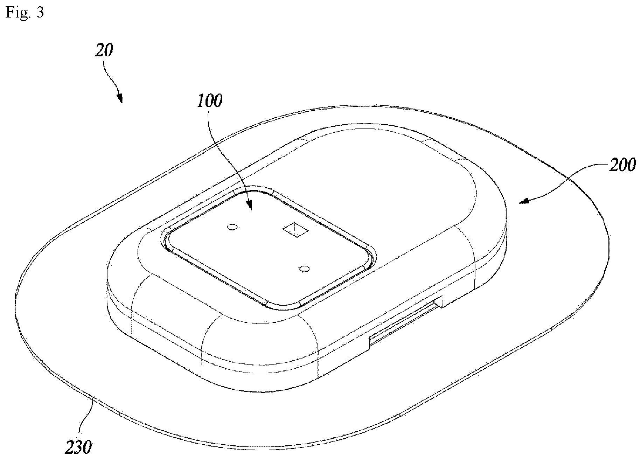

As shown in FIG. 3, the body attachable unit (20) includes the sensor unit (100) for measuring the biometric information of a user, and the base unit (200) that is attached to the skin of a user and coupled to the sensor unit (100). do. The base unit (200) may have electronic components installed therein. This base unit (200) is electrically connected to the sensor unit (100) and can process biometric information measured by the sensor unit (100) and transmit it to the external terminal (5).

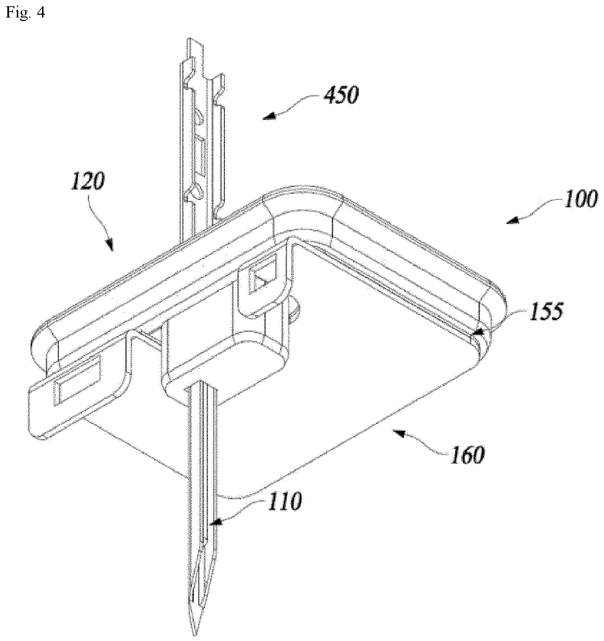

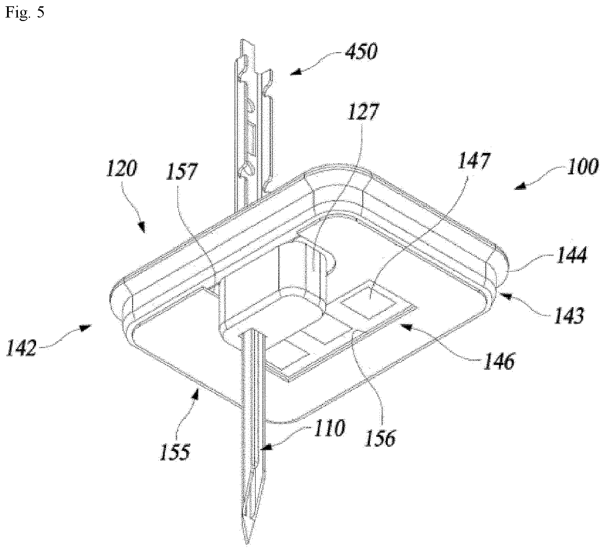

As shown in FIGS. 4 to 6, the sensor unit (100) includes a sensor (110) inserted into the skin of a user, a sensor unit housing (120) to which the sensor (110) is coupled, a sensor unit-electrical contact portion (146) coupled to the unit housing (120) and electrically connected to the sensor (110), an adhesive layer (155) provided on the sensor unit housing (120) to be attached to the base unit (200) and protective sheet (160) covering the adhesive layer (155).

A portion of the sensor (110) is disposed inside the sensor housing (120) and is electrically connected to the sensor unit-electrical contact portion (146). The sensor (110) has an insertion portion (116) that protrudes from the sensor housing (120) and can be inserted into the skin of a user.

In the sensor unit housing (120), a housing opening to which a needle (450) for inserting the sensor (110) or a part of the sensor (110) into the skin of a user is inserted is formed to penetrate the sensor unit housing (120) in the thickness direction. The sensor unit housing (120) includes a housing body (142) and a boss (127) protruding from one side of the housing body (142). The housing opening is formed to penetrate the housing body (142) and the boss (127). The housing body (142) is shaped to fit into the base unit recess (213) of the base unit (200). The housing body (142) includes a body portion (143) provided with a boss (127) and a cover portion (144) that is wider than the body portion (143). The sensor unit housing (120) is not limited to the configuration shown, and may be changed to various other configurations on which the sensor (110) may be mounted and coupled to the base unit (200).

The sensor unit-electrical contact portion (146) is disposed in the sensor unit housing (120) to be electrically connected to the sensor (110) . The sensor unit-electrical contact portion (146) may include a plurality of terminal portions (147) for transmitting electrical signals. A portion of the sensor unit-electrical contact portion (146) is exposed to the surface of the sensor unit housing (120) so that it electrically connects the sensor (110) and the base unit-electrical contact portion (225) of the base unit (200). The sensor unit-electrical contact portion (146) can be omitted, and in this case, the base unit-electrical contact portion (225) may be electrically connected to the sensor (110) by directly contacting the sensor (110).

An adhesive layer (155) is disposed on the surface of the housing base (120). The adhesive layer (155) has adhesive properties on both sides. One side of the adhesive layer (155) is adhered to the surface of the sensor unit housing (120), and the other side of the adhesive layer (155) is covered with the protective sheet (160). The adhesive layer (155) may be attached to the base unit (200) after the protective sheet (160) is separated. An adhesive layer hole (156) is formed in the middle portion of the adhesive layer (155) to penetrate the adhesive layer (155) in the thickness direction. The sensor unit-electrical contact portion (146) may contact the base unit-electrical contact portion (225) of the base unit (200) through the adhesive layer hole (156). Therefore, the adhesive layer (155) seals between the sensor unit-electrical contact portion (146) and the base unit-electrical contact portion (225), thereby preventing moisture or foreign substances from entering the electrical connection portion between the sensor unit (100) and the base unit (200). An adhesive layer opening (157) is formed on one side of the adhesive layer (155) to penetrate the adhesive layer (155) in the thickness direction. The adhesive layer (155) is attached to the housing base (121) such that the sensor unit-electrical contact portion (146) is located inside the adhesive layer hole (156) and the boss (127) is inserted into the adhesive layer opening (157).

The protective sheet (160) covers and protects the adhesive layer (155). The protective sheet (160) is made of a material that is detachably attached to the adhesive layer (155). If the adhesive layer (155) is left exposed to the air for a long time, the adhesiveness of the adhesive layer (155) may deteriorate. The protective sheet (160) covers the adhesive layer (155) to prevent the problem of reducing of adhesiveness of the adhesive layer (155), and it facilitates handling of the sensor unit (100) by an operator during the manufacturing process of the sensor unit (100) or the process of assembling the sensor unit (100) to the applicator (30). A portion of the protective sheet (160) may be coupled with the removing unit (500) of the applicator (30).

The sensor unit (100) is mounted on the applicator (30) with the protective sheet (160) attached to the adhesive layer (155). The protective sheet (160) may be separated from the adhesive layer (155) by a removing unit (600) of the applicator (30) before the sensor (110) is inserted into the skin of a user. The sensor unit (100) moves toward the base unit (200) with the protective sheet (160) separated and is coupled to the base unit (200).

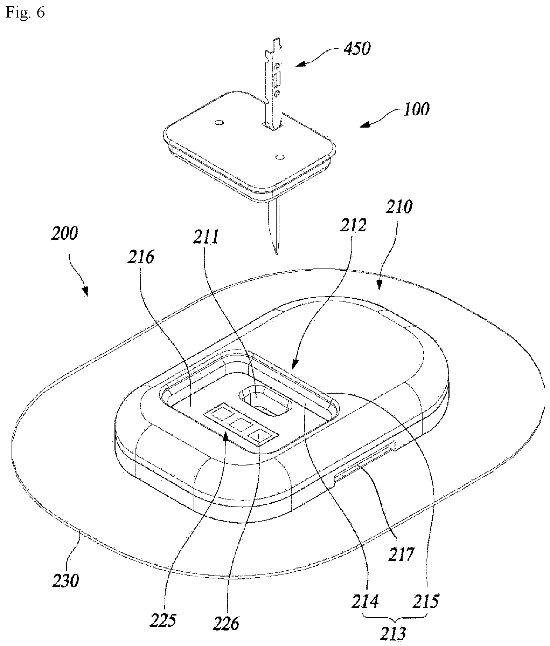

As shown in FIG. 6, the base unit (200) includes a base unit housing (210) to which the sensor unit (100) is coupled, and electronic components installed inside the base unit housing (210). The electronic component may include the base unit electrical contact portion (225) that contacts the sensor unit-electrical contact (146) of the sensor unit (100), a board, a battery, a processor chip for processing signals and a communication chip for wireless communication with the external terminal (5), etc.

The base unit housing (210) is provided with an insertion hole (211) through which the insertion portion (116) of the sensor (110) and the needle (450) can pass, and a mounting portion (212) in which the sensor unit housing (120) is coupled. The mounting portion (212) includes a base unit recess (213) and a contact surface (216) provided inside the base unit recess (213). The insertion hole (211) is shaped so that the boss (127) of the sensor unit (100) can be fitted and is disposed inside the base unit recess (213). The contact surface (216) may be flat so that the adhesive layer (155) of the sensor unit (100) can be stably adhered. The base unit recess (213) includes a first recess (214) connected to the insertion hole (211), and a second recess (215) that is located farther from the insertion hole (211) than the first recess (214) and is connected to the first recess (214). The second recess (215) is wider than the first recess (214). The first recess (214) can be made in a shape corresponding to the body portion (143) of the sensor unit housing (120), and the second recess (215) can be made in a shape corresponding to the cover portion (144) of the sensor unit housing (120). Therefore, the sensor unit housing (120) can be fitted into the base unit recess (213) and maintain a stable coupling state with the base unit housing (210). In addition, since the sensor unit housing (120) is stably fitted and coupled to the base unit housing (210), moisture or foreign substances cannot easily enter the gap between the sensor unit housing (120) and the base unit housing (210). A housing groove (217) is formed on the outer edge of the base unit housing (210). Some part of a locking hook (360) provided on the applicator (30) may be inserted into the housing groove (217).

The base unit-electrical contact portion (225) is arranged on the mounting portion (212) and contacts the sensor unit-electrical contact portion (146) of sensor unit (100) when sensor unit (100) is coupled to the base unit (200). The base unit electrical-contact portion (225) is installed in the base unit housing (210) so that a portion is exposed into the base unit recess (213). The base unit-electrical contact portion (225) may include a plurality of terminal portions (226) for transmitting electrical signals. The terminal portion (226) may electrically connect the sensor unit-electrical contact portion (146) and a circuit board (223) by contacting the terminal portion (147) of the sensor unit (100).

In order to electrically connect the sensor (110) and the base unit (200), the specific configuration, number, and location of the base unit-electrical contacts portion provided in the base unit (200) may be changed in various ways. Additionally, the configuration for electrically connecting the sensor (110) and the base unit (200) is not limited to that shown and may vary in various ways.

An adhesive portion (230) is provided on the surface of the base unit housing (210). The adhesive portion (230) may be attached to the surface of the lower housing (219) to adhere the base unit housing (210) to the skin of a user. The adhesive portion (230) may be covered with a protective sheet and protected. The protective sheet covering the adhesive portion (230) may be removed in the process of attaching the base unit (200) to the skin of a user.

The sensor unit (100) and the base unit (200) are installed on the applicator (30) in a separated state, and in the process in which the applicator (30) is operated to insert the sensor (110) into the skin of user, they are coupled to each other and then form a body attachable unit. The sensor unit (100) may be mounted on the applicator (30) in a state in which the needle (450) is coupled, and moved toward the base unit (200) with the needle (450) to be coupled to the base unit (200). After the sensor unit (100) is coupled to the base unit (200), the needle (450) is separated from the sensor unit (100), and only the body attachable unit (20) remains on the skin of a user.

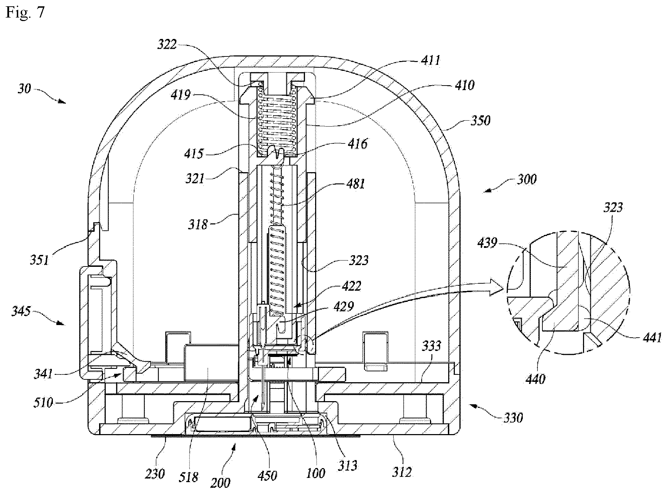

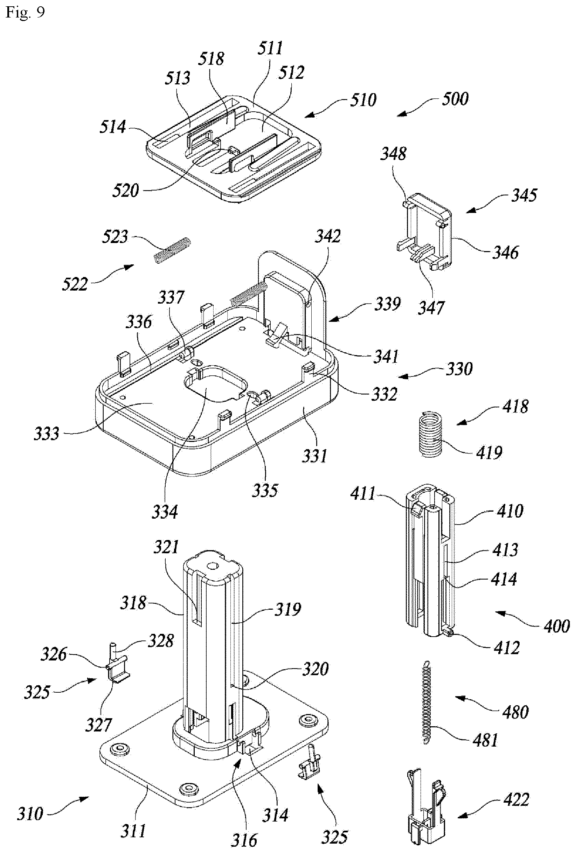

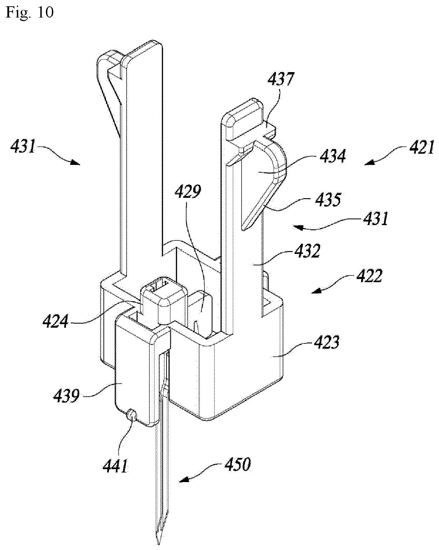

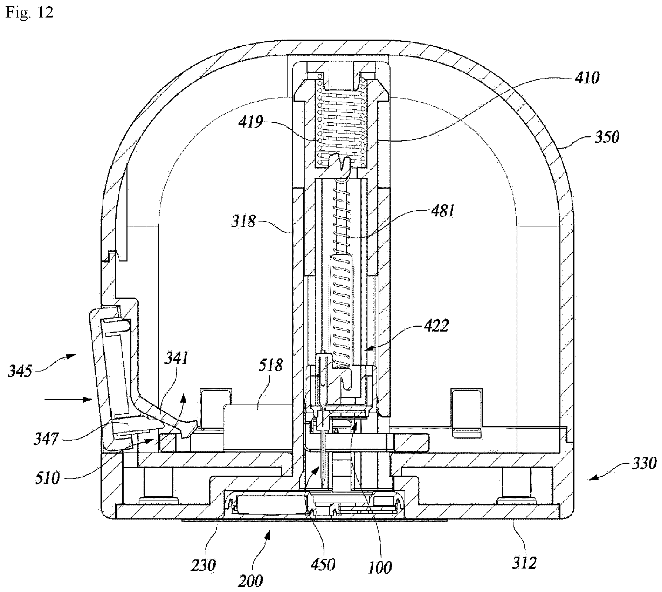

Referring to FIGS. 7 to 19, the applicator (30) operates with the sensor unit (100) and the base unit (200) mounted, thereby coupling the sensor unit (100) and the base unit (200) and can attach the body attachable unit (20) in which the sensor unit (100) and the base unit (200) are coupled to the skin of a user. The sensor unit (100) and the base unit (200) are spaced apart from each other and are respectively mounted on the applicator (30). The applicator (30) is separated from the body attachable unit (20) after attaching the body attachable unit (20) to the skin of a user, and the body attachable unit (20) remains attached to the skin of a user by the adhesive portion (230).

The applicator (30) includes an applicator body (300) to which the base unit (200) is detachably coupled, an insertion unit (400) that moves the sensor unit (100) to insert the sensor (110) into the skin of a user, and removing unit (500) for removing the protective sheet (160) of the sensor unit (100). The insertion unit (400) may move the sensor unit (100) from a first position spaced apart from the base unit (200) by a preset distance to a second position coupled with the base unit (200). The sensor unit (100) and a top case (350) which is coupled to the middle frame (330) and covers the upper part of the middle frame (330).

The applicator body (300) includes a base frame (310) on which the insertion unit (400) is installed, a middle frame (330) disposed on the base frame (310) and on which the removing unit (500) is installed, and a top case (350) that is coupled to and covers the upper part of the middle frame (330).

The base frame (310) includes a frame base portion (311) having a bottom portion (312) that can be in contact with the skin of a user, and a column portion (318) which protrudes from the frame base portion (311) and accommodates the sensor unit (100) and the insertion unit (400). The bottom portion (312) of the frame base portion (311) is provided with a recess (313) into which the base unit (200) is mounted. The base unit (200) can be detachably coupled to the recess (313) so that the adhesive portion (230) can face the skin of a user and placed in a second position spaced apart from the sensor unit (100). A through hole (314) is formed in the frame base portion (311) to penetrate the frame base portion (311) in the thickness direction. A pair of through holes (314) are provided on both sides of the column portion (318) with the column portion (318) interposed therebetween. A support portion (316) to which the locking hook (325) is coupled is provided at the top of the through hole (314).

Slits (319) are formed on both sides of the column portion (318) in a direction parallel to the moving direction of the sensor unit (100). A release portion (320) is provided in the middle of the slit (319). The release portion (320) is a portion of the carrier (422) that can be contacted while the carrier (422) of the insertion unit (400) moves toward the base unit (200). The carrier (422) may be disengaged from the plunger (410) by the action of the release portion (320). The specific operation of the release portion (320) will be described later. A detent portion (321) is provided on one side of the column portion (318) to limit the moving distance of the plunger (410). A retention unit (322) is provided inside the column unit (318). The retention unit (322) serves to support the plunger driver (418), which provides moving force to the plunger (410).

A locking hook (325) engaged with the base unit (200) mounted in the recess (313) is coupled to the support portion (316) of the base frame (310). The locking hook (325) engages the base unit (200) and fixes the base unit (200) so as not to be separated from the recess (313). The locking hook (325) may be disengaged from the base unit (200) after the body attachable unit (20) is attached to the skin of a user. The locking hook (325) is pivotally coupled to the support portion (316). The locking hook (325) includes a pivot portion (326) rotatably coupled to the support portion (316), a hook portion (327) connected to the pivot portion (326), and a rod (328) protruding from the pivot portion (326). The hook portion (327) may be inserted into the housing groove (217) of the base unit (200) and engaged with the base unit (200). The hook portion (327) protrudes from one side of the pivot portion (326), and the rod (328) protrudes from the other side of the pivot portion (326). The hook portion (327) is inserted into the through hole (314) of the base frame (310), and a portion of it is located in the recess (313) of the base frame (310). The locking hook (325) may be disengaged from the base unit (200) in conjunction with the moving member (510) of the removing unit (500). A more specific operation of the locking hook (325) will be described later.

The specific configuration of the base frame (310) is not limited to that shown and can be changed in various ways.

The middle frame (330) includes an outer frame (331) coupled to the base frame (310), a stage (333) disposed inside the outer frame (331), and a support fixture (339) protruding from the stage (333). The outer frame (331) is provided with a hook portion (332) that can be engaged with the top case (370). The stage (333) is disposed on the frame base portion (311). A middle frame opening (334) into which the column portion (318) is inserted is provided in the middle of the stage (333). On both sides of the middle frame opening (334), a through hole (335) is formed into which the rod (363) of the locking hook (360) is inserted. The rod (363) may pass through the through hole (335) and protrude onto the stage (333). The stage (333) is provided with a guide rail (336) and a fixing protrusion (337). The guide rail (336) serves to guide the moving member (510) to move linearly. The fixing protrusion (337) supports the moving member driver (522) that provides moving force to the moving member (510). The support fixture (339) is provided with an elastically deformable stopper (341) for restraining the movement of the moving member (510).

An operating member (345) capable of operating the insertion unit (400) and the removing unit (500) is installed on the support fixture (339). The operating member (345) includes a button portion (346), a pressing protrusion (347) protruding from the button portion (346) so as to contact the stopper (341), and a pivot portion (348) inserted into the coupling hole (342) of the support (339). The operating member (345) may rotate around the pivot portion (348). When a user presses the button portion (346), the operating member (345) rotates so that the pressing protrusion (347) presses the stopper (341). At this time, the stopper (341) may be elastically deformed and disengaged from the moving member (510). In addition to the configuration shown, the operating member (345) can be changed to various different configurations in which it is installed on the applicator body (300) to be operated by a user and can operate the insertion unit (400) and the removing unit (500).

The specific configuration of the middle frame (330) is not limited to that shown and can be changed in various ways. As another exemplary embodiment, the middle frame (330) may be formed integrally with the base frame (310).

The top case (350) is coupled with the middle frame (330) and covers the upper portions of the middle frame (330) and the base frame (310). On one side of the top case (350), a top case opening (351) is provided in a shape which allows the support fixture (339) of the middle frame

(330) to be coupled. The top case (350) has a coupling hole (352) and can be coupled to the middle frame (330) by engaging the hook portion (332) of the middle frame (330) with the coupling hole

(352).

The specific configuration of the top case (350) is not limited to that shown and can be changed in various ways. Additionally, the method of coupling the top case (350) and the middle frame (330) can also be changed in various ways.

The insertion unit (400) is installed on the applicator body (300) to associate with the moving member (510) of the removing unit (500) to move the sensor unit (100) from the first position to the second position, and the sensor (110) can be inserted into the skin of a user. The insertion unit (400) includes a plunger (410) movably installed on the middle frame (330) and a needle assembly (421) having a needle (450) that can be moved with the plunger (410) and inserted into the skin of a user.

The plunger (410) is installed inside the column portion (318) to be movable from the first position to the second position. A plunger hook (411) is provided on one side of the plunger (410) to limit the movement range of the plunger (410). The plunger hook (411) protrudes from the plunger (410) and can limit the movement of the plunger (410) by contacting the detent portion (321) of the column portion (318) when the plunger (410) moves to the second position. A plunger protrusion (412) is provided on the other side of the plunger (410). The plunger protrusion (412) protrudes from the plunger (410) to engage with the moving member (510). The plunger (410) is fixed in the first position when the plunger protrusion (412) is engaged with the moving member (510), and can be moved to the second position when the plunger protrusion (412) is disengaged from the moving member (510).

A pair of slits (413) are formed on both sides of the plunger (410) parallel to the moving direction of the sensor unit (100). A detent portion (414) with which the latch portion (437) of the carrier (422) can be engaged is provided in the middle portion of the slit (413). In a state where the latch portion (437) is engaged with the detent portion (414), the carrier (422) moves together with the plunger (410) and cannot move relative to the plunger (410).

A retention portion (415) and a fixing portion (416) are provided inside the plunger (410). The retention portion (415) supports the plunger driver (418). A needle release driver (480) that provides a moving force to the carrier (422) is coupled to the fixing portion (416).

The plunger (410) moves by a moving force from the plunger driver (418). The plunger driver (418) includes an elastic member (419) that applies elastic force to the plunger (410) in a direction of moving from the first position to the second position. The elastic member (419) has one end in contact with the retention portion (322) of the column portion (318) and the other end in contact with the retention portion (415) of the plunger (410). The plunger (410) may be fixed in the first position by engaging the moving member (510) in a state in which the elastic member (419) is elastically deformed. The plunger driver (418) may be changed to various other configurations that can provide a moving force to the plunger (410) in addition to the configuration including the elastic member (419) in the form of a coil spring.

The needle assembly (421) includes the carrier (422) to which the sensor unit (100) is detachably coupled, and a needle (450) coupled to the carrier (422) penetrating the sensor unit (100). In a state in which the needle (450) is coupled to the sensor unit (100), the needle (450) advance from the first position to the second position together with the sensor unit (100) to be inserted into the skin of a user, and then may retreat with the carrier (422) in the second position and be separated from the skin of a user and the sensor unit (100).

The carrier (422) is coupled to the plunger (410) to enable relative movement. A portion of the carrier (422) engages the plunger (410) so as to move with the plunger (410) from the first position to the second position. Then, the carrier (422) is disengaged from the plunger (410) in the second position and may retreat in a direction away from the sensor unit (100).

The carrier (422) includes a carrier body (423) to which the needle (450) is coupled, an elastically deformable carrier wing (431) extending from the carrier body (423), and a locking arm (439) connected to the side of the carrier body (423).

The carrier body (423) includes a boss (424) with an insertion groove (425) formed on the inside. A portion of the needle (450) may be inserted into the insertion groove (425). The needle (450) may be inserted into the insertion groove and fixed to the carrier body (423). various shapes of clamp portions that engage with the needle (450) may be provided in the insertion groove.

The needle release driver (480) provides a moving force to the carrier (422). The needle release driver (480) includes an elastic member (481) whose one end is coupled to the fixing portion (416) of the plunger (410) and the other end is coupled to the fixing portion (429) of the carrier (422). The elastic member (481) is in the form of a tension spring and applies an elastic force to pull the carrier (422) toward the fixing portion (416) of the plunger (410). That is, the elastic member (481) can apply an elastic force to the carrier (422) in a direction away from the sensor unit (100) to move the needle (450) coupled to the carrier (422) to be separated from the sensor unit (100). The needle release driver (480) may be changed to various other configurations that can provide a moving force to the carrier (422) in addition to the configuration including the elastic member (481) in the form of a coil spring.

The carrier wing (431) is connected to the carrier body (423) in a shape that extends from the carrier body (423) in the moving direction of the carrier (422). The carrier wing (431) includes a wing body (432) elastically deformably connected to the carrier body (423), and a trigger (434) and a latch portion (437) protruding from the wing body 432.

The trigger (434) is provided on one side of the wing body (432) to protrude outward from the wing body (432). The trigger (434) is inserted into the slit (413) of the plunger (410) and the slit (319) of the column portion (318) and can move along these slits (413) and (319). The trigger (434) is inserted into the slits (413 and 319) and guided by the slits (413 and 319), so that the carrier (422) can move linearly more stably. The trigger (434) passes through the slit (413) of the plunger (410), and some part is inserted into the slit (319) of the column portion (318). While the carrier (422) moves with the plunger (410) from the first position to the second position, the trigger (434) may contact the release portion (320) disposed in the middle of the slit (319) of the column portion (318).

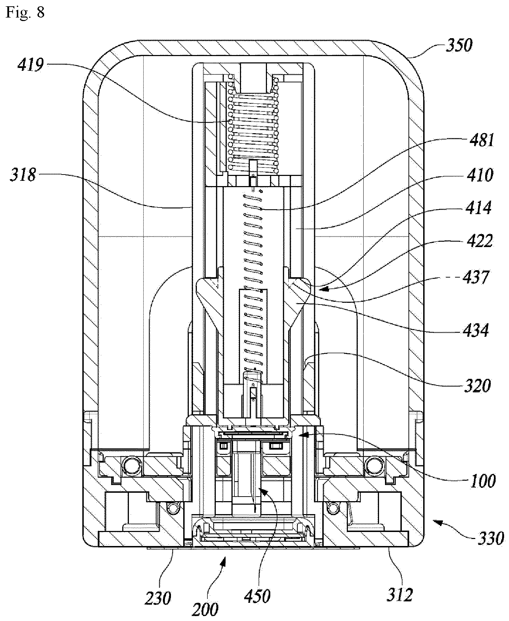

As shown in FIG. 8, the release portion (320) is disposed in the middle of the slit (319) of the column portion (318) to press the trigger (434). While the carrier (422) is moving from the first position to the second position, the trigger (434) may come into contact with the release portion (320) and the carrier wing (431) may be elastically deformed. The trigger (434) is provided with a trigger inclined portion (435) that contacts the release portion (320). While the carrier (422) moves from the first position to the second position, the trigger inclined portion (435) comes into contact with the release portion (320), so that the carrier wing (431) can be elastically deformed more smoothly, and the impact that occurs when the trigger (434) comes into contact with the release portion (320) is reduced, and the pressing force of the release portion (320) can be more smoothly transmitted to the trigger (434).

The latch portion (437) protrudes outward from the wing body (432). The direction in which the latch portion (437) protrudes from the wing body (432) is the same as the direction in which the trigger (434) protrudes from the wing body (432). The protrusion height at which the latch portion (437) protrudes from the wing body (432) is lower than the protrusion height at which the trigger (434) protrudes from the wing body (432). In a state in which the latch portion (437) is engaged with the detent portion (414), the carrier (422) can maintain the elastic member (481) in an elastically deformed state, and cannot move in the direction in which the elastic force of the needle release driver (480) acts.

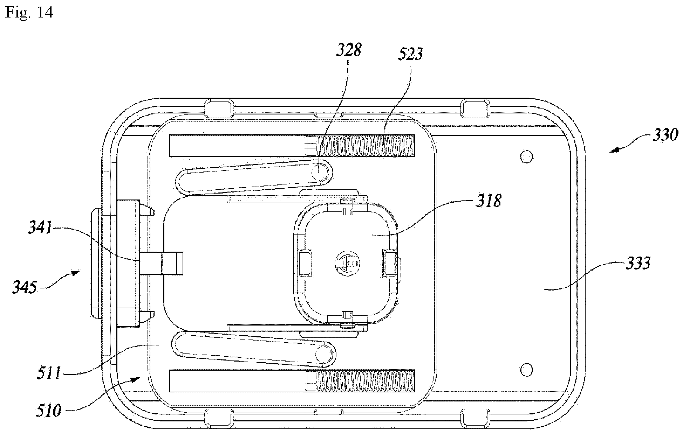

The locking arm (439) is provided on the side of the carrier body (423) to be elastically deformable. A pair of locking arms (439) are arranged to face each other on both sides of the carrier body (423) and detachably fix the sensor unit housing (120) of the sensor unit (100). The locking arm (439) is provided with a locking protrusion (440) engaged with the sensor unit housing (120) and a protrusion (441) in contact with an inner wall (323) of the column portion (318). As shown in FIG. 14, when the carrier (422) and the sensor unit (100) are located in the first position, as the protrusion (441) contacts the inner wall (323) of the column portion (318), the locking arm (439) is biased in a direction in which it engages with the sensor unit housing (120). Accordingly, the sensor unit (100) can remain stably coupled to the carrier (422) in the first position.

Meanwhile, when the sensor unit (100) and the carrier (422) move toward the base unit (200) and the sensor unit (100) is coupled with the base unit (200), the protrusion (441) is located in a space connected to the recess (313) in which the base unit (200) is accommodated and deviates from the inner wall (323) of the column portion (318). Therefore, when the sensor unit

(100) is coupled to the base unit (200), the fixing force of the locking arm (439) is weakened. And the sensor unit (100) is fixed to the base unit (200) by the adhesive layer (155), so that when the carrier (422) is pulled in a direction away from the body attachable unit (20) by the needle release driver (480), the locking arm (439) can be smoothly disengaged from the sensor unit (100) and separated from the sensor unit (100).

In addition to the configuration shown, the carrier (422) can be changed to various other configurations in which it is coupled to the needle (450) and installed to be relatively movable on the plunger (410). For example, the drawing shows a pair of carrier wings (431) being symmetrically provided on both sides of the carrier body (423), but the number and shape of the carrier wings (431) can be changed in various ways. Additionally, the coupling method of the carrier (422) and the needle (450) and the coupling method of the carrier (422) and the sensor unit (100) may also be changed in various ways.

The needle (450) is fixed to the carrier (422) and can move from the first position to the second position while coupled to the sensor unit (100). The needle (450) has a sharp tip so as to pierce the skin of a user and be smoothly inserted into the skin of a user. When the sensor unit (100) moves to the second position, the needle (450) penetrates the skin of a user before the sensor (110) and allows the sensor (110) to be stably inserted into the skin. The needle (450) is separated from the skin of a user after the sensor (110) is inserted into the skin of a user.

Before the sensor unit (100) reaches the second position and is coupled to the base unit (200), the removing unit (500) is installed on the applicator body (300) to separate the protective sheet (160) of the sensor unit (100) from the adhesive layer (155). The removing unit (500) includes a moving member (510) movably installed on the middle frame (330) to be coupled to the protective sheet (160) and a moving member driver (522) that moves the moving member (510) by providing a moving force to the moving member (510).

The moving member (510) is arranged to move linearly on the stage (333) of the middle frame (330). The moving member (510) is installed to move in a direction that is approximately perpendicular to the direction in which the sensor unit (100) moves from the first position to the second position. The moving member (510) may pull the protective sheet (160) to be separated from the adhesive layer (155) by moving while coupled with the protective sheet (160). Specifically, in a state in which the protective sheet (160) covers the adhesive layer (155), the moving member (510) can move from a third position where the protective sheet is coupled to a fourth position where the protective sheet (160) is removed from the adhesive layer. Additionally, the moving member (510) may act as a stopper that fixes the plunger (410) in the first position. That is, the moving member (510) fixes the plunger (410) at the first position by coming into contact with the plunger (410) located in the first position at the third position, and when the moving member (510) moves to the fourth position, it deviates from the plunger (410) so that the plunger (410) can move to the second position.

The moving member (510) includes a moving member body (511), a supporting portion (518) for supporting the plunger (410), and a grip portion (520) coupled with the protective sheet (160).

A moving member opening (512) and a moving member groove (513) are formed in the moving member body (511) to penetrate the moving member body (511) in the thickness direction. The moving member opening (512) is formed in the middle of the moving member body (511), and a pair of moving member grooves (513) are disposed on both sides of the moving member body (511). A fixing protrusion (514) connected to the moving member driver (522) is provided in the moving member groove (513). A rail portion (515) is provided on one surface of the moving member body (511) facing the stage (333) of the middle frame (330). The rail portion (515) may engage with the guide rail (336) of the middle frame (330) and slide along the guide rail (336). In the drawing, the guide rail (336) is shown in a groove shape and the rail portion (515) is inserted into the guide rail (336), but the guide rail (336) and the rail portion (515) can be changed into various other configuration that can guide the moving member body (511) to stably move linearly on the stage (333).

A pair of supporting portions (518) are disposed at both edges of the moving member opening (512) to face each other. The supporting portion (518) may protrude from the moving member body (511) and support the plunger protrusion (412) of the plunger (410). The length of the supporting portion (518) is shorter than the distance that the moving member body (511) moves from the third position to the fourth position. When the moving member (510) is located in the third position, the supporting portion (518) may support the plunger protrusion (412) of the plunger (410) and fix the plunger (410) in the first position. And when the moving member (510) moves to the fourth position, the supporting portion (518) deviates from the plunger protrusion (412) so that the plunger (410) can move to the second position. The supporting portion (518) may be changed into various other shapes that can support the plunger protrusion (412) or a portion of the plunger (410) in addition to the shape that protrudes from the moving member body (511).

The grip portion (520) is connected to the moving member body (511) so as to be located in the moving member opening (512). A portion of the grip portion (520) is inserted into a groove provided in the protective sheet (160) and is coupled to the protective sheet (160). The coupling method of the moving member (510) and the protective sheet (160) may be changed to various other methods other than using the grip portion (520). As another exemplary embodiment, the moving member (510) may be coupled to the protective sheet (160) through an adhesive method, or may be coupled to the protective sheet (160) by engaging an edge of the protective sheet (160).

The moving member driver (522) provides a moving force to the moving member (510) so that the moving member (510) can be moved from the third position to the fourth position. The moving member driver (522) includes an elastic member (523) that applies elastic force to the moving member (510) in a direction that is biased to the fourth position. The elastic member (523) is inserted into the moving member groove (513) of the moving member (510), so that one end is connected to the fixing protrusion (337) of the middle frame 330 and the other end is connected to the fixing protrusion (514) of the moving member (510). The moving member driver (522) may be changed into various other forms that can provide a moving force to the moving member (510) in addition to configuration including the elastic member (523) in the form of a coil spring.

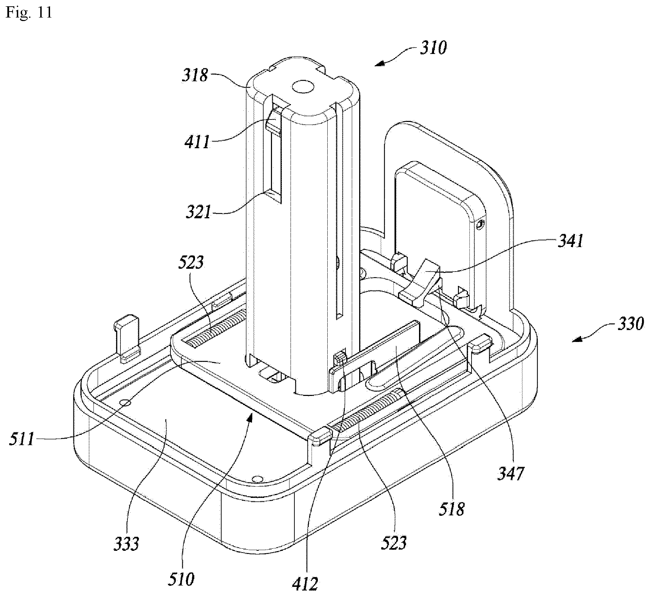

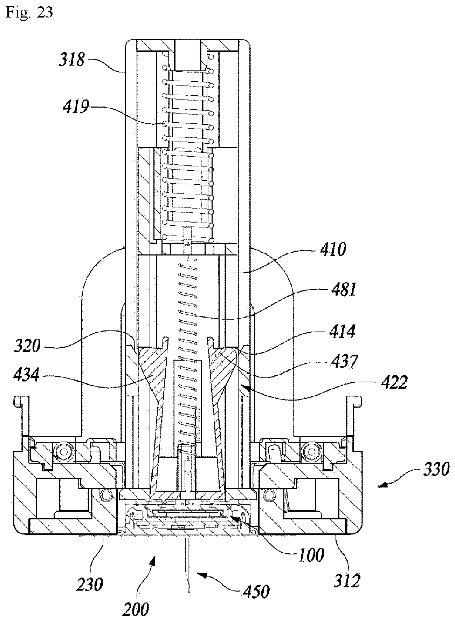

As shown in FIG. 11, the moving member (510) may engage the stopper (341) provided in the middle frame (330) at the third position and be fixed in a state in which the elastic member (523) is elastically deformed. A portion of the stopper (341) is inserted into the moving member opening (512) of the moving member (510) and engages with the moving member (510). The stopper (341) may be released by the operating member (345) to remove the binding force on the moving member (510). That is, as shown in FIG. 23, when a user presses the operating member (345), the operating member (345) can bias the stopper (341) to be disengaged from the moving member (510).

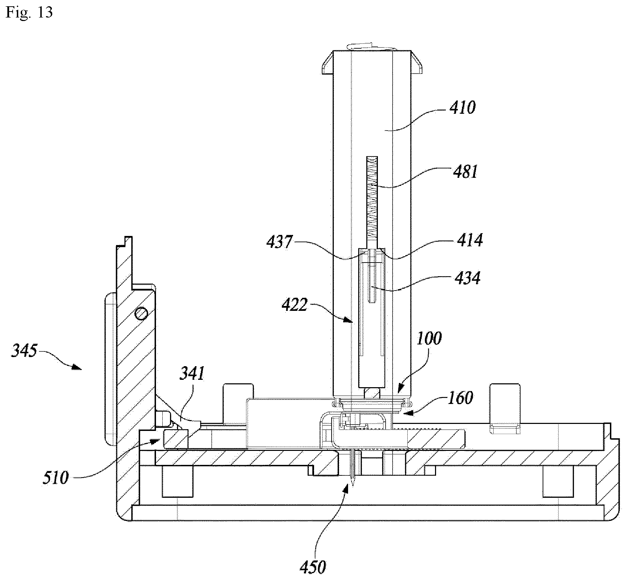

FIGS. 13 and 14 show a state in which the moving member (510) is engaged with the stopper (341) and fixed in the third position. At this time, the moving member (510) supports the plunger (410) and fixes the plunger (410) in the first position, and the protective sheet (160) coupled with the moving member (510) maintains the adhesive layer (155) covered.

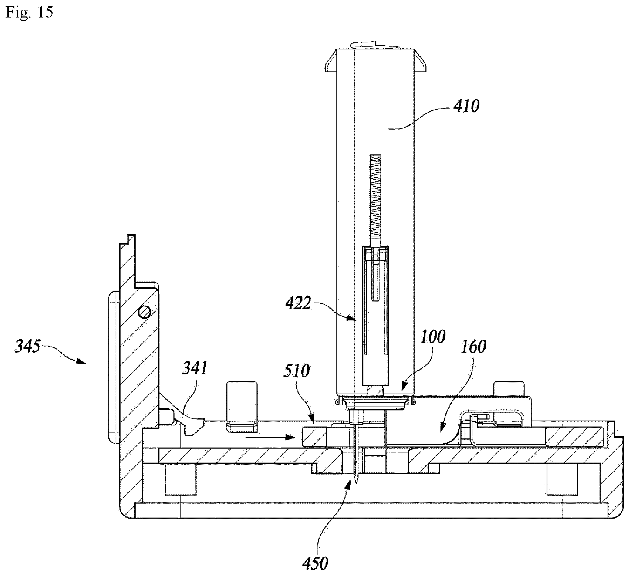

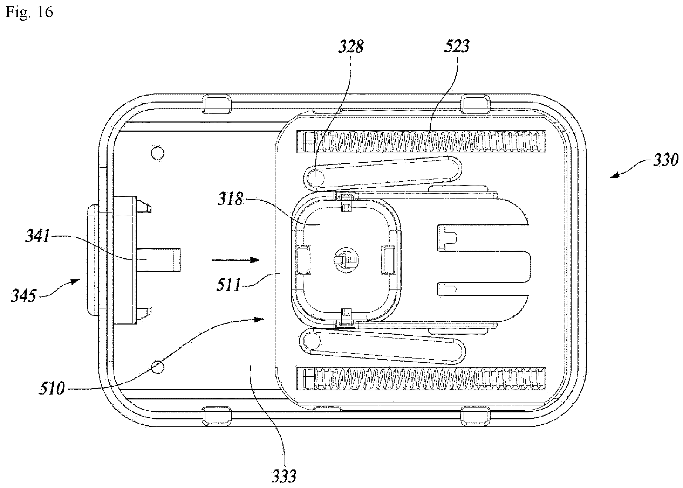

FIGS. 15 and 16 show the moving member (510) being disengaged from the stopper (341). At this time, the moving member (510) moves to the fourth position dragging the protective sheet (160). Accordingly, the protective sheet (160) is separated from the adhesive layer (155), and the plunger (410) can be moved out of the moving member (510) and moved to the second position by the plunger driver (418).

When the length of the support portion (518) supporting the plunger protrusion (412) of the plunger (410) is sufficiently long, the moving member (510) may be released from the plunger (410) after completely separating the protective sheet (160) from the adhesive layer (155). In this case, the separation operation of the protective sheet (160) is completed while the sensor unit (100) is located in the first position, and then the sensor unit (100) can be moved to the second position. Depending on the length of the supporting portion (518), it is possible that the plunger (410) starts to move toward the second position during the separation operation of the protective sheet (160).

In this way, the moving member (510) has the function of separating the protective sheet (160) and operating the insertion unit (400) in conjunction with the operating member (345). In addition to these functions, the moving member (510) can move the locking hook (325) that secures the base unit (200). For this purpose, a guide portion (516) engaged with the locking hook (325) is provided on one side of the moving member body (511) facing the stage (333) of the middle frame (330).

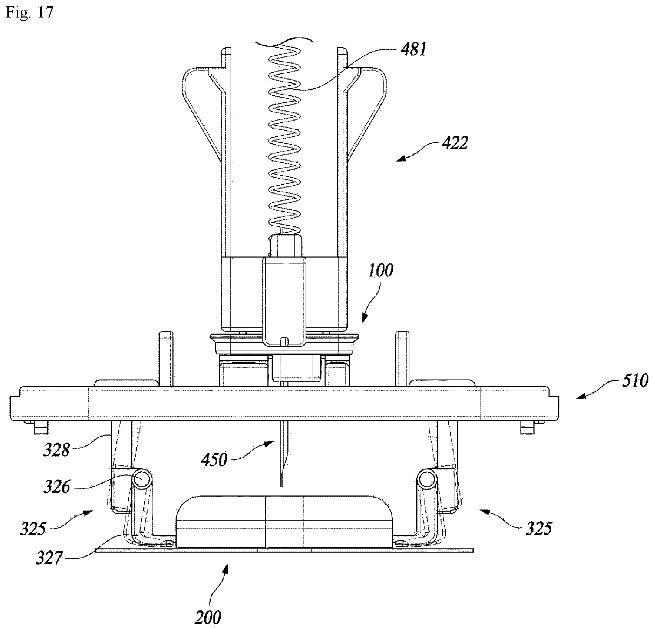

The guide portion (516) is formed in a groove shape that is disposed to be inclined with respect to the moving direction of the moving member (510) so that the end of a rod (328) of the locking hook (325) can be engaged. Since the end of the rod (328) is engaged with the guide portion (516), the locking hook (325) can rotate in conjunction with the moving member (510). As shown in FIG. 17, the locking hook (325) may rotate in the direction in which the hook portion (327) engages with the base unit (200) or rotate in a direction in which the hook portion (327) moves away from the base unit (200) depending on the position of the moving member (510).

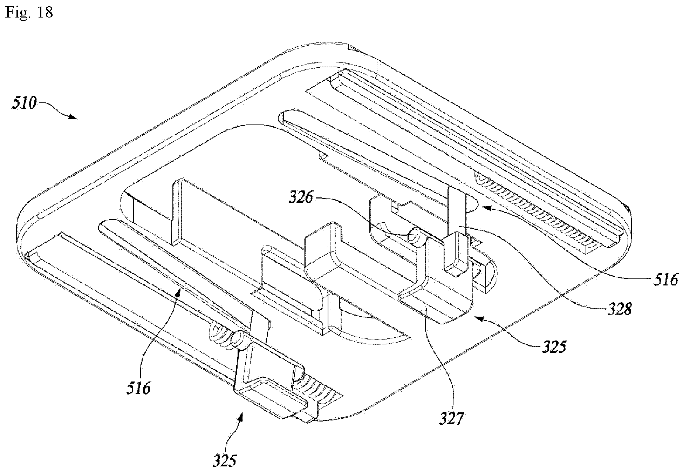

As shown in FIGS. 14 and 18, when the moving member (510) is located in the third position, the guide portion (516) biases the locking hook (325) in a direction in which the hook portion (327) engages with the base unit (200).

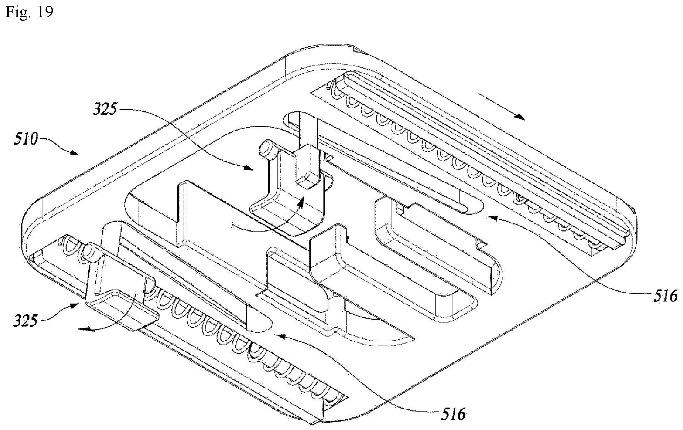

On the other hand, as shown in FIGS. 16 and 19, when the moving member (510) moves to the fourth position, the guide portion (516) rotates the locking hook (325) in a direction in which the hook unit (327) is disengaged from the base unit (200). That is, when the moving member (510) moves from the third position to the fourth position, the rod (328) of the locking hook (325) is pulled by the guide portion (516) in a direction approaching the moving member opening (512) of the moving member (510). At this time, by rotating around the pivot portion (326), the locking hook (325) is biased so that the hook portion (327) is disengaged from the base unit (200).

Due to the action of the movable member (510), when the movable member (510) is located in the third position, the locking hook (325) maintains a state of being stably engaged with the base unit (200) and may fix the base unit (200) so as not to be separated from the recess (313) of the applicator body (300) by maintaining a state in which the locking hook (325) is stably engaged with the base unit (200). And when the moving member (510) moves to the fourth position, the locking hook (325) is disengaged from the base unit (200) so that the base unit (200) can be separated from the applicator body (300). Therefore, after the sensor unit (100) is coupled with the base unit (200) and the body attachable unit (20) is assembled, the body attachable unit (20) can be smoothly separated from the applicator (30) while being attached to the skin of a user by the adhesive portion (230).

In the drawing, the guide portion (516) is shown to be in the form of a groove, but the guide portion is changed to various other shapes that engage the locking hook (325) and can bias the locking hook (325) according to the movement of the moving member (510). Depending on the shape of the guide portion, etc., the locking hook may also be changed into various other configurations that can be detachably engaged with the base unit (200).

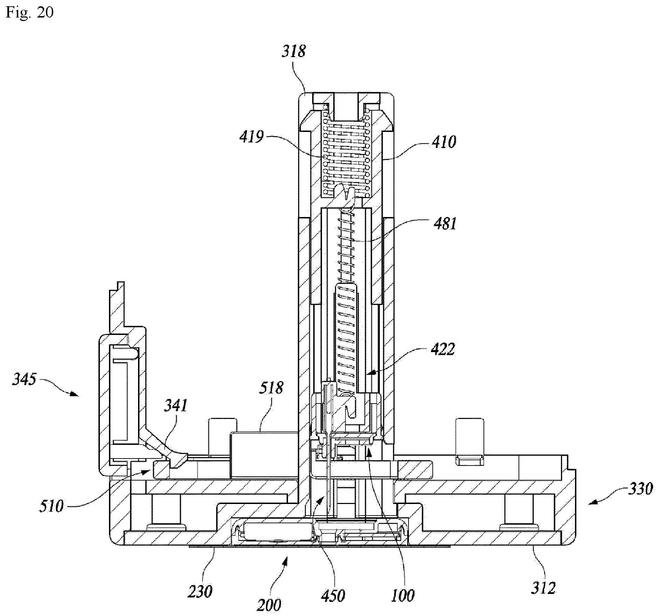

Hereinafter, the operation of the applicator assembly (10) according to an embodiment of the present disclosure will be described with reference to FIGS. 20 to 25.

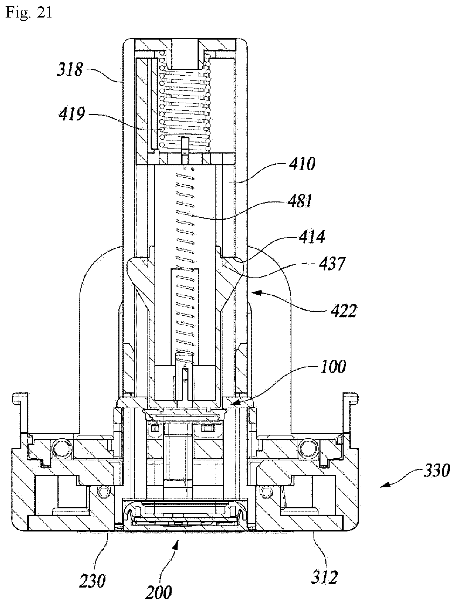

Referring to FIGS. 20 and 21, first, the bottom portion (312) of the applicator (30) is placed on the skin of a user so that the base unit (200) is attached to the skin of a user by the adhesive portion (230). Before the operating member (345) is manipulated, the sensor unit (100) coupled to the carrier (422) is positioned in the first position in a state in which the protective sheet (160) covers the adhesive layer (155). And the moving member (510) is located in the third position engaging with the stopper (341).

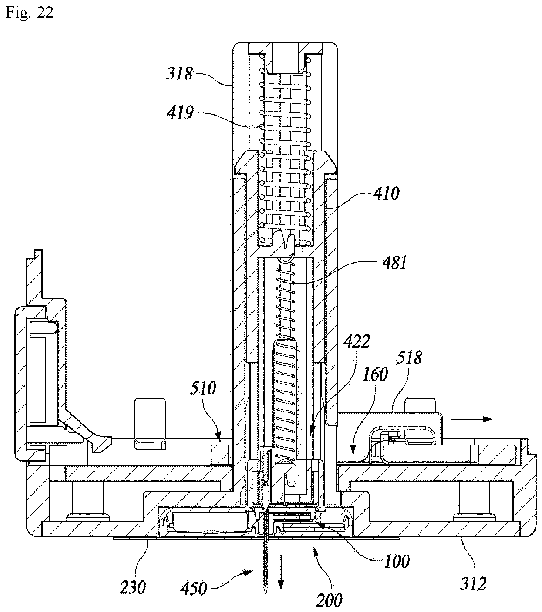

Thereafter, when the operating member (345) is pressed, the moving member (510) is disengaged from the stopper (341) and the moving member (510) is moved to the fourth position by the moving member driver (522). As shown in FIGS. 22 and 23, as the moving member (510) moves to the fourth position, the protective sheet (160) is pulled by the moving member (510) and separated from the adhesive layer (155). Then, the plunger (410) moves away from the moving member (510) and moves to the second position by the plunger driver (418). At this time, the insertion portion (116) of the sensor (110) and needle (450) are inserted into the skin of a user, and the sensor unit (100) from which the protective sheet (160) is separated is attached to the base unit (200) by the adhesive layer (155), so that the body attachable unit (20) is assembled.

When the plunger (410) moves and the insertion portion (116) and the needle (450) are inserted into the skin of a user, the trigger (434) of the carrier (422) comes into contact with the release portion (320) of the column portion (318). At this time, the carrier wing (431) of the carrier (422) is elastically deformed, so that the latch portion (437) of the carrier (422) is disengaged from the detent portion (414) of the plunger (410).

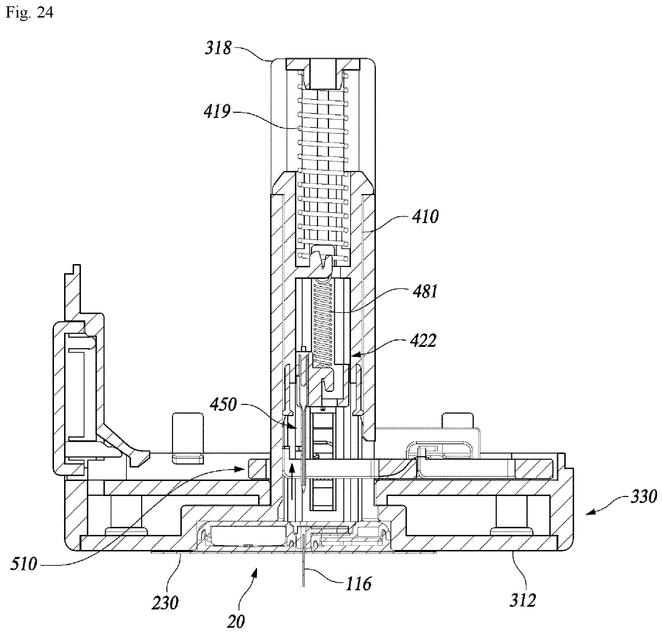

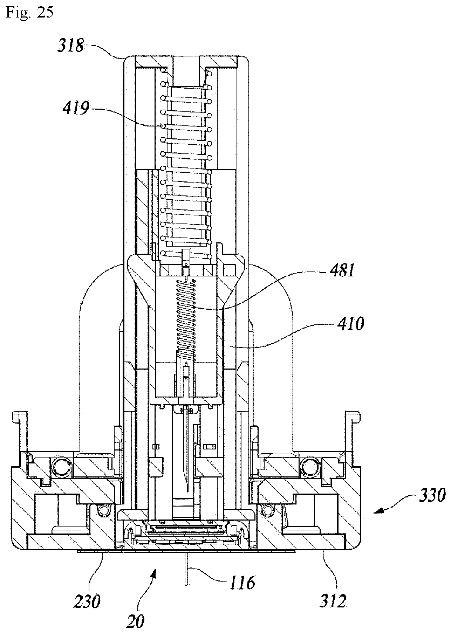

When the carrier (422) is disengaged from the plunger (410), the carrier (422) moves away from the skin of a user by the elastic member (481) of the needle release driver (480) as shown in FIGS. 24 and 25. At this time, the needle (450) comes out of the skin of a user and the body attachable unit (20) remains attached to the skin of a user by the adhesive portion (230).

In a state in which the sensor unit (100) is coupled with the base unit (200) and the body attachable unit (20) is assembled, the locking hook (325) engaged with the base unit (200) rotates in conjunction with the moving member (510). That is, the locking hook (325) is biased to be disengaged from the base unit (200) by the moving member (510). Accordingly, the body attachable unit (20) can be separated from the applicator (30) while being attached to the skin of a user.

The body attachable unit (20) attached to the skin of a user and separated from the applicator (30) can measure biometric information and transmit the measurement information to an external terminal (5) and the like.

As described above, the applicator assembly (10) according to an embodiment of the present disclosure can remain stably coupled to the applicator body (300) so that the base unit (200), which is assembled with the sensor unit (100) to form a body attachable unit (20), is spaced apart from the sensor unit (100) by the locking hook (325). In addition, in the process in which the sensor unit (100) moves and then is coupled with the base unit (200), the locking hook (325) is disengaged from the base unit (200), so that the base unit (200) can be smoothly separated from the applicator (30).

Although the present disclosure has been described above with preferred examples, the scope of the present disclosure is not limited to the configurations described and shown above.

For example, the drawing shows that the moving member for moving the locking hook (325) is a component of a removing unit for separating the protection sheet from the sensor unit, but the moving member for moving the locking hook (325) may be a component provided separately from the removing unit.

Additionally, the drawing shows that the moving member is installed to move in a direction that intersects approximately perpendicular to the moving direction of the sensor unit, but the moving member may be installed to move in another direction.

In addition, the drawing shows that in a state in which the elastic member that provides the moving force to the moving member is elastically deformed, a stopper that restrains the movement of the moving member is provided in the middle frame, but the position of the stopper can be changed in various ways. As another exemplary embodiment, the stopper may be installed at various locations on the applicator body other than the middle frame. As another exemplary embodiment, the stopper is provided on a moving member to engage with the applicator body and can be released by the operating member.

In addition, the drawing shows that the sensor unit is detachably coupled to the carrier coupled with the needle and moves from the first position to the second position, but the sensor unit is detachably coupled to the plunger or a separate member provided on the plunger and can move together with the plunger.

Additionally, as another exemplary embodiment, the base unit may simply take a configuration to support the sensor unit so that it is not separated from the skin of a user. In this case, a separate electronic unit that can process biometric information measured by the sensor unit and transmit it to an external terminal may be detachably coupled to the base unit. A separate electronic unit may be coupled to the base unit to be electrically connected to the sensor unit after the sensor unit is coupled to the base unit.

Although the present disclosure has been shown and described in connection with preferred embodiments for illustrating the principles of the disclosure, the disclosure is not limited to the construction and operation as shown and described. Rather, those skilled in the art will understand that numerous changes and modifications can be made to the present disclosure without departing from the concept and scope of the attached registration claims.

## Claims

1. An applicator for inserting a sensor for measuring biometric information into skin of a user, the applicator comprising:
an applicator body to which a base unit comprising a base unit housing and an adhesive portion provided on the base unit housing to be attached to the skin of the user is separatably coupled;
a locking hook installed to the applicator body to be engaged with the base unit to maintain a state in which the base unit is coupled to the applicator body;
an insertion unit installed to the applicator body to insert the sensor into the skin of the user by moving the sensor unit, comprising the sensor and a sensor unit housing to which the sensor is mounted, from a first position spaced apart from the base unit to a second position coupled with the base unit; and
a moving member installed to the applicator body to be movable from a third position to a fourth position, and configured to bias the locking hook to be disengaged from the base unit at the fourth position.

2. The applicator according to claim 1,
wherein the moving member is installed to move in a direction of intersecting a direction in which the sensor unit moves from the first position to the second position.

3. The applicator according to claim 2, wherein
the insertion unit comprises
a plunger installed to be movable from the first position to the second position together with the sensor unit,
a needle separatably coupled with the sensor unit to be inserted into the skin of the user with the sensor,
a carrier installed to the plunger and coupled with the needle, and
a plunger driver configured to provide a moving force to the plunger in a direction of moving from the first position to the second position, and
the plunger is configured to be restricted from moving at the first position by contacting the moving member located at the third position, and, when the moving member moves to the fourth position, move away from the moving member and move to the second position.

4. The applicator according to claim 3, wherein:
the applicator body comprises
a base frame comprising a base portion configured to be contactable with the skin of the user, and a column portion protruding from the base portion and accommodating the plunger, and
a middle frame having a stage disposed at an upper side of the base portion to support the moving member, and a middle frame opening formed in a middle of the stage such that the column portion is inserted in the middle frame opening.

5. The applicator according to claim 4, further comprising:
an elastic member configured to apply an elastic force to the moving member such that the moving member is movable from the third position to the fourth position;
a stopper configured to restrain movement of the moving member in a state in which the elastic member is elastically deformed; and
an operating member configured to release the stopper such that the moving member is movable by the elastic member according to manipulation of the user.

6. The applicator according to claim 1,
wherein the locking hook is pivotally coupled to the applicator body, and the moving member moves from the third position to the fourth position to rotate the locking hook in a direction of disengaging the locking hook from the base unit.

7. The applicator according to claim 6,
wherein the locking hook comprises
a pivot portion coupled to the applicator body,
a hook portion protruding from the pivot portion to be engaged with the base unit, and
a rod protruding from the pivot portion to be engaged with the moving member,
wherein the moving member is installed to move in a direction of intersecting a direction in which the sensor unit moves from the first position to the second position, and
wherein the moving member is provided with a guide portion engaged with the rod in an inclined form with respect to a moving direction of the moving member, and the guide portion moves in a state of being engaged with the rod to rotate the locking hook.

8. The applicator according to claim 1, further comprising
a moving member driver configured to move the moving member from the third position to the fourth position.

9. The applicator according to claim 8,
wherein the moving member driver includes an elastic member configured to apply an elastic force to the moving member.

10. The applicator according to claim 9, further comprising:
a stopper configured to restrain movement of the moving member in a state in which the elastic member is elastically deformed; and
an operating member configured to release the stopper such that the moving member is movable by the elastic member according to manipulation of the user.

11. An applicator for inserting a sensor for measuring biometric information into skin of a user, the applicator comprising:
an applicator body to which a body attachable unit base unit comprising the sensor, a base unit housing, and an adhesive portion provided on the base unit housing to be attached to the skin of the user is separatably coupled;
a locking hook installed to the applicator body to be engaged with the base unit housing to maintain a state in which the base unit housing is coupled to the applicator body;
a needle installed to the applicator body such that the needle is movable from the first position to the second position to be inserted into the skin of the user together with the sensor; and
a moving member installed to the applicator body to be movable from a third position to a fourth position, and configured to bias the locking hook to be disengaged from the base unit housing at the fourth position.

12. An applicator assembly comprising:
an applicator body configured to be contactable with skin of a user;
a sensor unit comprising a sensor and a sensor unit housing to which the sensor is mounted;
a base unit including a base unit housing and an adhesive portion provided on the base unit housing to be attached to the skin of the user, disposed to be spaced apart from the sensor unit, and separatably coupled to the applicator body;
an insertion unit installed to the applicator body to move the sensor unit from a first position away from the base unit to a second position where the sensor is inserted into the skin of the user to couple the sensor unit with the base unit;
a locking hook installed to the applicator body to be engaged with the base unit to maintain a state in which the base unit is coupled to the applicator body; and
a moving member installed to the applicator body to be movable from a third position to a fourth position, and configured to bias the locking hook to be disengaged from the base unit at the fourth position.
